# EUROPEAN PATENT APPLICATION

(11) **EP 4 417 598 A1**
(43) Date of publication of application: **21.08.2024**
(21) Application number: 23156957.5
(22) Date of filing: 16.02.2023
(51) Int. Cl.: C07C 231/02, C07C 233/20, C07C 233/18, C07C 233/08

(54) **A PROCESS FOR THE PREPARATION OF CERAMIDES**

(71) Applicant: Roelmi HPC S.r.l, 21040 Origgio (VA) (IT)
(72) Inventor: PANZA, Luigi, 13100 VERCELLI (VC) (IT); IMPERIO, Daniela, 13100 VERCELLI (VC) (IT); ALBEVERIO, Filippo, 13100 VERCELLI (VC) (IT); CARLOMAGNO, Federica, 21040 ORIGGIO (VA) (IT); CRIMALDI, Antonio, 21040 ORIGGIO (VA) (IT); MALANCHIN, Rosella, 21040 ORIGGIO (VA) (IT)
(74) Representative: Bianchetti & Minoja with Trevisan & Cuonzo IPS SRL

(57) **Abstract**

The present invention provides a novel and environmentally sustainable synthesis method applicable to production of several ceramides in good yield and high purity. The invention method is efficient, selective, and can be carried out on a large scale for industrial production of ceramides.

## Description

### Field of the invention

The present invention provides an improved process for the preparation of ceramides by selective acylation of phyto-sphingosine.

### Background of the Invention

Ceramides are molecules naturally present in large quantities in the human body. They belong to the class of sphingolipids and are composed mainly by sphingosine, phyto-sphingosine or 6-hydroxysphingosine. These one, are C₁₈-spingoid bases that through amide linkage with a variety of non-hydroxy or hydroxy acids can form different type of ceramides. Ceramides are involved as a second messenger in various cellular events, including proliferation, differentiation, cell cycle arrest, apoptosis and senescence. Following this, it is clear that there is a correlation between ceramides levels and cancer pathogenesis. Indeed, in a variety of studies, has been observed like alterations in ceramide's metabolism are involved in controlling cancer initiation, progression and response of cancer cells to chemotherapeutic agents and radiation. Endogeneous levels of ceramides are suppressed in many types of tumor tissues compared to non-pathological condition. Instead, exposure of cancer cells to a variety of stress agents as cytokines, anticancer drugs and radiation cause an increase of ceramides levels, which promote tumor death. The process of tumor death is regulated by various mechanisms, such as apoptosis, necroptosis, autophagy and mitophagy. In addition, ceramides are showing a lot of interest in cosmetic and dermatological fields. Skin consists of three layers, the epidermis, dermis and the hypodermis. The epidermis consists of several layers: stratum basale, stratum spinosum, stratum granulosum, stratum lucidum and stratum corneum. The stratum corneum is the outermost layer of the skin and it is composed of dead keratinocytes and three main lipid components: ceramides, cholesterol and fatty acids. Ceramides play an important role in structuring and maintaining the water permeability barrier function of the skin, contribute to the solubility of cholesterol, reducing fluidity of biological membranes, and affects skin cell regulation and signaling. In fact, it has been observed that decreased levels of these molecules within the intercellular lipid lamellae of the stratum corneum is associated to psoriasis, atopic dermatitis and dry skin.

Ceramides and their derivatives such as cerebrosides, gangliosides and sphingomyelins are therefore of commercial and therapeutic importance. Ceramides, for example, are of great commercial prospective in cosmetics and pharmaceuticals e.g. in hair and skin care products.

As regards the synthesis of ceramides, the selective acylation of the amino group in amino alcohols like sphingosine remains a challenge and simple, selective, and inexpensive methods for their production are still needed.

Ceramides are typically obtained by extraction from natural sources, typically from bovine brain or porcine epidermal tissues, with consequent high cost, uneven purity and concerns for the presence of animal contaminants.

Many methods are known for the acylation of amino groups of the starting material. However, most of them are described on simple substrates having single functional groups and are hardly applicable for the preparation of multifunctional compounds such as ceramides. Other methods exploit condensing agents, which are expensive and furthermore render difficult the product purification.

Moreover, in most of the methods known, the formation of esters on the hydroxyl groups of the amino alcohol (O-acylation) cannot be avoided, thus leading to side reactions involving undesired, and sometimes even toxic, by-products, thus reducing the yield and the purity of the desired product. In fact, it is known that acylation of sphingosine and sphinganine with acylating agents, such as acyl chlorides, leads to the formation of N- and O-acylated products (Ong, D. and Brody, R. J. Lipid Res., 1972, 13, 819-822), requiring an additional step for the removal of the O-acyl groups, thus not leading to a selective acylation.

Recent methods of production of sphingolipids are disclosed in WO 9320038 A1, where it is described the production of N-acyl amino alcohols via the selective acylation of the free amines of amino alcohols with acyl-sulfonyl mixed anhydrides, particularly using p-toluene-sulfonyl chloride (TsCl) as acylating agent. Nevertheless, the presence of the sulfonic-carboxylic anhydrides as intermediate is undesirable, also due to the toxicity of the compound.

Hence, it is still felt the need to obtain an efficient, fast, simple, non-toxic and sustainable method to synthesize ceramides by selective N-acylation of a sphingoid base.

### Description of the Invention

In a first aspect, the invention provides a process for the preparation of ceramides comprising the steps of:
a) reacting pivaloyl chloride in the presence of a base and an organic solvent with an aliphatic acid of formula (I):

   R-COOH (I),

   or a salt therefor, wherein R is a straight or a branched C₇-C₂₅ alkyl chain optionally interrupted by an oxygen atom, optionally containing one or more double bonds, and optionally being substituted with one or more hydroxyl groups, thus obtaining a mixed anhydride;
b) reacting said mixed anhydride with phyto-sphingosine, thus obtaining a ceramide.

According to the invention, the aliphatic acids, or their salts, of the formula R-COOH (I) react with pivaloyl chloride, in the presence of organic or inorganic bases, to form the mixed anhydride. The so formed mixed anhydride is thus allowed to react with a sphingoid base, namely the phyto-sphingosine, always in the presence of a base.

The whole reaction process can be carried out "one-pot", by first preparing the mixed anhydride followed by addition of sphingosine to the reaction medium, without the necessity to isolate the mixed anhydride intermediate. The process of the invention thus requires only a simple, inexpensive and convenient set-up, wherein the reagent do not need to be moved in different flasks or reactors, thus avoiding materials loss and reaction yield reduction.

In one preferred aspect of the present invention, the organic aliphatic acids used are saturated, mono- or poly-unsaturated, linear or branched C₈-C₂₆ fatty acids.

Preferably, the aliphatic acid of formula (I) is selected from the group consisting of oleic acid, palmitic acid, stearic acid, pelargonic acid, cerotic acid and 12-hydroxystearic acid.

According to the invention, the selectivity of N-acylation allows the hydroxyl groups of the aliphatic acid, if present, to remain unprotected, thus avoiding further steps of protection and deprotection.

In one embodiment of the invention, the base of steps a) and b) is an organic base, preferably selected from the group consisting of tertiary amines, more preferably it is triethyl amine, pyridine, and mixtures thereof.

In another embodiment of the present invention, the base of steps a) and b) is an inorganic base, preferably selected from the group consisting of cesium carbonate, potassium carbonate and mixtures thereof.

The organic solvents used in steps a) and b) are the same or different and preferably comprise or consist of dichloromethane, ethyl acetate, tetrahydrofuran, methyl tetrahydrofuran, toluene, chloroform, methyl ethyl ketone, diethyl ether and mixtures thereof. In a preferred embodiment, the organic solvent is selected from tetrahydrofuran and methyl tetrahydrofuran.

Likewise preferred for its lower environmental impact is 2-methyl tetrahydrofuran.

In a preferred embodiment, in step a) the ratio of the aliphatic acid of formula (I) to pivaloyl chloride is 1:1.

In another preferred embodiment, in step a) the ratio of the organic base to pivaloyl chloride is in the range from 2:1 to 3:1, more preferably it is 2.2:1.

The reaction for the formation of the mixed anhydride in step a) is carried out at a temperature ranging from 0 to 30°C, preferably from 0 to 5°C.

The reaction for the formation of the mixed anhydride in step a) is preferably carried out for a time ranging from 1 to 2 hours, more preferably 1.5 hours.

In one embodiment, the desired ceramide in step b) is prepared using a slight excess of mixed anhydride with respect to the amino alcohol and preferably the ratio of the mixed anhydride to phyto-sphingosine ranges from 1:0.7 to 1.5:1, more preferably 1:0.95.

These conditions, in addition to the selectivity of the reagents and the specific synthetic steps, prevent the formation of esters through O-acylation of the hydroxyl group on the phyto-sphingosine, thus avoiding the formation of undesired by-products which affect the purity of the final ceramide.

The reaction of the mixed anhydride with phyto-sphingosine in step b) is preferably carried out at a temperature ranging from 0 to 30°C, preferably from 20 to 28°C.

Advantageously, step b) is carried out for a time ranging from 1 to 8 hours, preferably 3 hours.

The reaction can be monitored by means such as thin layer chromatography (TLC) to determine the presence of the residues of phyto-sphingosine and mixed anhydride starting materials, as well as that of the ceramide final product.

At the end of the reaction, the solvent is removed and the product is taken up with water/solvent mixtures. Suitable water/solvent mixtures include water/acetone, preferably in a 9:1 ratio. In a preferred embodiment, the precipitate is filtered off and rinsed using water/acetone mixtures combined with acetone or isopropyl alcohol washing steps.

Further steps for the purification of the ceramide can be carried out to eliminate traces of O-acylated by-products.

As it will be evident from the experimental part, the process of the present invention produces the desired ceramides from phyto-sphingosine in good yields, with only traces of concomitant O-acylated derivatives.

### Experimental Part

### Example 1: Synthesis of N-(1,3,4-trihydroxyoctadecan-2-yl)oleamide

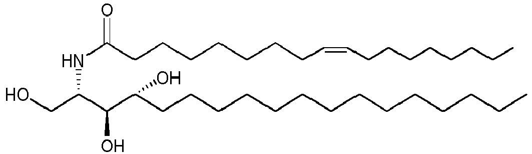

A solution of oleic acid (1 g, 3.54 mmol) in 2 mL of tetrahydrofuran and triethyl amine (1.09 mL, 7.79 mmol) is added dropwise to a stirred solution of pivaloyl chloride (0.43 g, 3.54 mmol) in 13 mL of tetrahydrofuran at 0°C. After stirring for 90 minutes at 0°C and monitoring by TLC that the mixed anhydride is formed, the reaction mixture is heated at room temperature. After that, phyto-sphingosine (1.07 g, 3.36 mmol) and other 5 mL of tetrahydrofuran is added, and the reaction is stirred overnight at room temperature. 300 mL of water is added and stirring is continued for 1 hour. After evaporation of tetrahydrofuran, 20 mL of a solution of water/acetone 9:1 is added. The precipitate is filtered off using a sintered glass filter and washed twice, each one with 20 mL water/acetone 9:1 and other twice with 15 mL acetone for each one. Drying gives 1.50 g of pure compound. Yield 77%.

¹H NMR (400 MHz, CDCl₃/MeOD 5:1) δ 5.32 - 5.29 (m, 2H, vinyl H), 4.04 (br m, 1H, H2), 3.75 (dd, J = 11.4, 4.1 Hz, 1H, H1a), 3.64 (dd, J = 11.5, 5.3 Hz, 1H, H1b), 3.50 (br m, 2H, H3 and H4), 2.19 (t, J = 7.4 Hz, 2H, H2'), 2.00 (m, 4H, allylic H), 1.64 - 1.22 (m, 48H, CH₂), 0.85 (t, J = 6.6 Hz, 6H, 2 CH₃)

¹³C NMR (101 MHz, CDCl₃/MeOD 5:1) δ 174.55, 129.93, 129.64, 75.66, 72.42, 61.00, 51.87, 36.47, 33.02, 31.85, 31.82, 29.62, 29.58, 29.44, 29.28, 29.23, 27.12, 27.10, 25.79, 25.71, 22.59, 13.94.

### Example 2: Synthesis of N-(1,3,4-trihydroxyoctadecan-2-yl)palmitamide

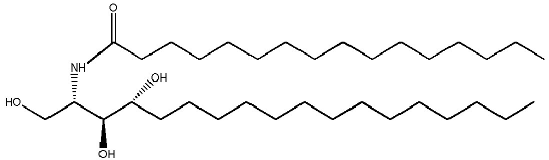

A solution of palmitic acid (0.91 g, 3.54 mmol) in 5 mL of tetrahydrofuran and triethyl amine (1.09 mL, 7.79 mmol) is added dropwise to a stirred solution of pivaloyl chloride (0.43 g, 3.54 mmol) in 10 mL of tetrahydrofuran at 0°C. After stirring for 1 hour at 0°C and monitoring by TLC that the mixed anhydride is formed, the reaction mixture is heated at room temperature. After that, phyto-sphingosine (1.07 g, 3.36 mmol) and other 17 mL of tetrahydrofuran is added, and the reaction is stirred overnight at room temperature. 300 mL of water is added and stirring is continued for 1 hour. After evaporation of tetrahydrofuran, 20 mL of a solution of water/acetone 9:1 is added. The precipitate is filtered off using a sintered glass filter and washed twice, each one with 30 mL water/acetone 9:1 and other twice with 20 mL acetone for each one. Drying gives 1.59 g of pure compound. Yield 85%.

¹H NMR (400 MHz, CDCl₃/MeOD 5:1) δ 4.04 (br m, 1H, H2), 3.75 (dd, J = 11.5, 3.7 Hz, 1H, H1a), 3.64 (dd, J = 11.5, 5.4 Hz, 1H, H1b), 3.50 (br m, 2H, H3 and H4), 2.17 (t, J = 7.4 Hz, 2H, H2'), 1.67 - 1.22 (m, 52H, CH₂), 0.85 (t, J = 6.5 Hz, 6H, 2 CH₃).

¹³C NMR (101 MHz, CDCl₃/MeOD 5:1) δ 174.61, 75.67, 72.41, 61.02, 51.88, 36.49, 33.01, 31.84, 29.61, 29.57, 29.44, 29.28, 29.22, 25.77, 25.72, 22.59, 13.94.

### Example 3: Synthesis of N-(1,3,4-trihydroxyoctadecan-2-yl)stearamide

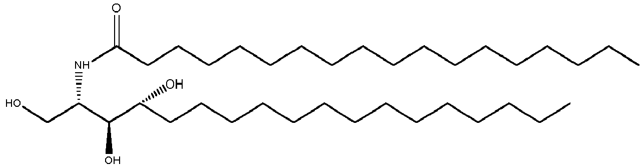

A solution of stearic acid (1.01 g, 3.54 mmol) in 8 mL of tetrahydrofuran and triethyl amine (1.09 mL, 7.79 mmol) is added dropwise to a stirred solution of pivaloyl chloride (0.43 g, 3.54 mmol) in 12 mL of tetrahydrofuran at 0°C. After stirring for 50 minutes at 0°C and monitoring by TLC that the mixed anhydride is formed, the reaction mixture is heated at room temperature. After that, phyto-sphingosine (1.07 g, 3.36 mmol) and other 20 mL of tetrahydrofuran is added, and the reaction is stirred overnight at room temperature. 300 mL of water is added and stirring is continued for 1 hour. After evaporation of tetrahydrofuran, 20 mL of a solution of water/acetone 9:1 is added. The precipitate is filtered off using a sintered glass filter and washed twice, each one with 30 mL water/acetone 9:1 and other twice with 30 mL acetone for each one. Drying gives 1.69 g of pure compound. Yield 86%.

¹H NMR (400 MHz,CDCl₃/MeOD 5:1) δ 4.03 (br m, 1H, H2), 3.77 (dd, J = 11.5, 3.8 Hz, 1H, H1a), 3.64 (dd, J = 11.5, 5.4 Hz, 1H, H1b), 3.50 (br m, 2H, H3 and H4), 2.16 (t, J = 7.9 Hz, 2H, H2'), 1.67 - 1.22 (m, 56H, CH₂), 0.84 (t, J = 7.0 Hz, 6H, 2 CH₃).

¹³C NMR (101 MHz, CDCl₃/MeOD 5:1) δ 174.60, 75.68, 72.42, 61.01, 51.88, 36.49, 33.02, 31.84, 29.62, 29.57, 29.44, 29.28, 29.22, 25.77, 25.72, 22.59, 13.94

### Example 4: Synthesis of N-(1,3,4-tryhydroxyoctadecan-2-yl)nonamide (FA-42)

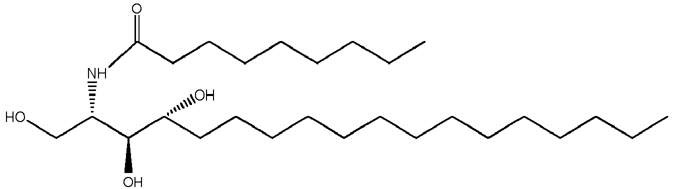

A solution of pelargonic acid (0.56 g, 3.54 mmol) in 5 mL of tetrahydrofuran and triethyl amine (1.09 mL, 7.79 mmol) is added dropwise to a stirred solution of pivaloyl chloride (0.43 g, 3.54 mmol) in 10 mL of tetrahydrofuran at 0°C. After stirring for 1 hour at 0°C and monitoring by TLC that the mixed anhydride is formed, the reaction mixture is heated at room temperature. After that, phyto-sphingosine (1.07 g, 3.36 mmol) and other 5 mL of tetrahydrofuran is added, and the reaction is stirred overnight at room temperature. 300 mL of water is added, and stirring is continued for 1 hour. After evaporation of tetrahydrofuran, 20 mL of a solution of water/acetone 9:1 is added. The precipitate is filtered off using a sintered glass filter and washed twice, each one with 20 mL water/acetone 9:1 and other twice with 15 mL acetone for each one. Drying gives 1.08 g of pure compound. Yield 70.2%.

¹H NMR (400 MHz, CDCl₃/MeOD 5:1) δ 4.04 (br m, 1H, H2), 3.75 (dd, J = 11.4, 3.7 Hz, 1H, H1a), 3.64 (dd, J = 11.5, 5.4 Hz, 1H, H1b), 3.49 (br m, 2H, H3 and H4), 2.17 (t, J = 7.6 Hz, 2H, H2'), 1.67 - 1.22 (m, 38H, CH₂), 0.84 (t, J = 6.7 Hz, 6H, 2 CH₃).

¹³C NMR (101 MHz, CDCl₃/MeOD 5:1) δ 174.59, 75.65, 72.42, 61.01, 51.87, 36.48, 33.01, 31.84, 31.75, 29.62, 29.58, 29.28, 29.24, 29.21, 29.08, 25.79, 25.71, 22.59, 22.55, 13.94, 13.91.

### Example 5: Synthesis of N-(1,3,4-trihydroxyoctadecan-2-yl)hexacosanamide

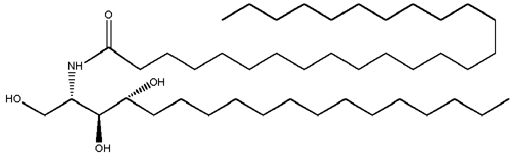

A solution of cerotic acid (0.70 g, 1.77 mmol) in 9 mL of tetrahydrofuran and triethyl amine (0.54 mL, 3.89 mmol) is added dropwise to a stirred solution of pivaloyl chloride (0.21 g, 1.77 mmol) in 10 mL of tetrahydrofuran at 0°C. After stirring for 1 hour at 0°C and monitoring by TLC that the mixed anhydride is formed, the reaction mixture is heated at room temperature. After that, phyto-sphingosine (0.53 g, 1.68 mmol) and other 27 mL of tetrahydrofuran is added, and the reaction is stirred overnight at room temperature. 150 mL of water is added and stirring is continued for 1 hour. After evaporation of tetrahydrofuran, 20 mL of a solution of water/acetone 9:1 is added. The precipitate is filtered off using a sintered glass filter and washed twice, each one with 20 mL water/acetone 9:1 and other twice with 15 mL acetone for each one. Drying gives 1.04 g of pure compound. Yield 89%.

¹H NMR (400 MHz, CDCl₃/MeOD 5:1) δ 4.05 (br m, 1H, H2), 3.76 (dd, J = 11.4, 4.0 Hz, 1H, H1a), 3.67 (dd, J = 11.4, 5.2 Hz, 1H, H1b), 3.52 (br m, 2H, H3 and H4), 2.19 (t, J = 7.6 Hz, 2H, H2'), 1.68 - 1.23 (m, 72H, CH2), 0.85 (t, J = 6.7 Hz, 6H, 2 CH₃).

¹³C NMR (101 MHz, CDCl₃/MeOD 5:1) δ 174.61, 75.66, 72.41, 61.02, 51.88, 36.48, 33.00, 31.84, 29.61, 29.58, 29.45, 29.30, 29.28, 29.22, 25.77, 25.72, 22.59, 13.94.

### Example 6: Synthesis of N-(1,3,4-trihydroxyoctadecan-2-yl)oleamide

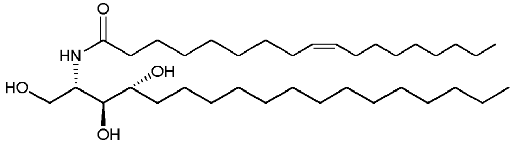

To a stirred solution at room temperature of potassium carbonate (0.98 g, 7.08 mmol), water (3 mL), cyclohexane (10 mL) and oleic acid (0.5g, 1.77 mmol) is added dropwise pivaloyl chloride (0.21 g, 1.77 mmol). After 10 minutes is added phyto-sphingosine (0.53 g, 1.68 mmol) and the reaction mixture is stirred overnight at room temperature. After that, 12 mL of hydrochloric acid 1 N is added. After evaporation of cyclohexane, the precipitate is filtered off using sintered glass filter and washed three times, each one with 20 mL water/acetone 9:1 and other three times with mL acetone for each one. Drying gives 0.64g of almost pure compound. Yield 65%.

¹H NMR (400 MHz, CDCl₃/MeOD 5:1) δ 5.32 - 5.29 (m, 2H, vinyl H), 4.04 (br m, 1H, H2), 3.75 (dd, J = 11.4, 4.1 Hz, 1H, H1a), 3.64 (dd, J = 11.5, 5.3 Hz, 1H, H1b), 3.50 (br m, 2H, H3 and H4), 2.19 (t, J = 7.4 Hz, 2H, H2'), 2.00 (m, 4H, allylic H), 1.64 - 1.22 (m, 48H, CH₂), 0.85 (t, J = 6.6 Hz, 6H, 2 CH₃)

¹³C NMR (101 MHz, CDCl₃/MeOD 5:1) δ 174.55, 129.93, 129.64, 75.66, 72.42, 61.00, 51.87, 36.47, 33.02, 31.85, 31.82, 29.62, 29.58, 29.44, 29.28, 29.23, 27.12, 27.10, 25.79, 25.71, 22.59, 13.94

### Example 7: Synthesis of 12-hydroxy-(N-1,3,4-trihydroxyoctadecan-2-yl)octadecanamide

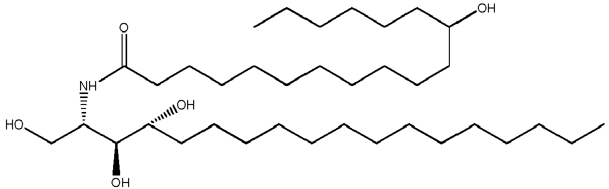

A solution of 12-hydroxystearic acid (1.06 g, 3.54 mmol) in 6 mL of tetrahydrofuran and triethyl amine (1.09 mL, 7.79 mmol) is added dropwise to a stirred solution of pivaloyl chloride (0.43 g, 3.54 mmol) in 13 mL of tetrahydrofuran at 0°C. After stirring for 1 hour at 0°C and monitoring by TLC that the mixed anhydride is formed, the reaction mixture is heated at room temperature. After that, phyto-sphingosine (1.07 g, 3.36 mmol) and other 31 mL of tetrahydrofuran is added, and the reaction is stirred overnight at room temperature. 300 mL of water is added and stirring is continued for 1 hour. After evaporation of tetrahydrofuran, 20 mL of a solution of water/acetone 9:1 is added. The precipitate is filtered off using a sintered glass filter and washed twice, each one with 20 mL water/acetone 9:1 and other twice with 20 mL acetone for each one. Drying gives 1.550 g of pure compound. Yield 77%.

¹H NMR (400 MHz, CDCl₃/MeOD 5:1) δ 4.04 (br m, 1H, H2), 3.74 (dd, J = 11.4, 3.7 Hz, 1H, H1a), 3.65 (dd, J = 11.4, 5.3 Hz, 1H, H1b), 3.50 (br m, 2H, H3 and H4), 2.18 (t, J = 7.6 Hz, 2H, H2'), 1.67 - 1.22 (m, 55H, CH₂), 0.84 (t, J = 6.6 Hz, 6H, 2 CH₃).

¹³C NMR (101 MHz, CDCl₃/MeOD 5:1) δ 174.55, 75.71, 72.43, 71.70, 61.03, 51.88, 37.19, 37.16, 36.45, 33.05, 31.83, 31.76, 29.60, 29.44, 29.36, 29.31, 29.29, 29.26, 29.17, 29.10, 25.75, 25.65, 25.50, 22.57, 22.52, 13.91, 13.87.

### Example 8: Synthesis of N-(1,3,4-trihydroxyoctadecan-2-yl)nonanamide

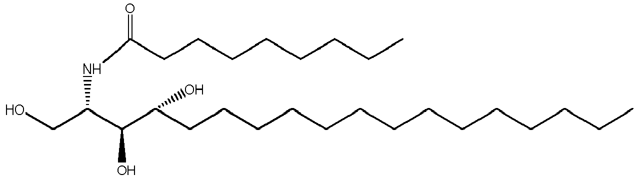

A solution of pelargonic acid (5.6 g, 35.4 mmol) in 50 mL of tetrahydrofuran and triethyl amine (10.9 mL, 77.9 mmol) is added dropwise to a stirred solution of pivaloyl chloride (4.27 g, 35.4 mmol) in 100 mL of tetrahydrofuran at 0°C. After stirring for 1.20 hour at 0°C and monitored by TLC that the mixed anhydride is formed, the reaction mixture is heated at room temperature. After that, phyto-sphingosine (10.7 g, 33.6 mmol) and other 50 mL of tetrahydrofuran is added, and the reaction is stirred overnight at room temperature. 3 mL of water is added and stirring is continued for 1 hour. After evaporation of tetrahydrofuran, 200 mL of a solution of water/acetone 9:1 is added. The precipitate is filtered off using a sintered glass filter and washed twice, each one with 200 mL water/acetone 9:1 and other twice with 150 mL acetone for each one. Drying gives 10.84 g of pure compound. Yield 70.5%

¹H NMR (400 MHz, CDCl₃/MeOD 5:1) δ 4.04 (br m, 1H, H2), 3.75 (dd, J = 11.4, 3.7 Hz, 1H, H1a), 3.64 (dd, J = 11.5, 5.4 Hz, 1H, H1b), 3.49 (br m, 2H, H3 and H4), 2.17 (t, J = 7.6 Hz, 2H, H2'), 1.67 - 1.22 (m, 38H, CH₂), 0.84 (t, J = 6.7 Hz, 6H, 2 CH₃).

¹³C NMR (101 MHz, CDCl₃/MeOD 5:1) δ 174.59, 75.65, 72.42, 61.01, 51.87, 36.48, 33.01, 31.84, 31.75, 29.62, 29.58, 29.28, 29.24, 29.21, 29.08, 25.79, 25.71, 22.59, 22.55, 13.94, 13.91.

### Example 9: Synthesis of N-(1,3,4-tryhydroxyoctadecan-2-yl)nonanamide

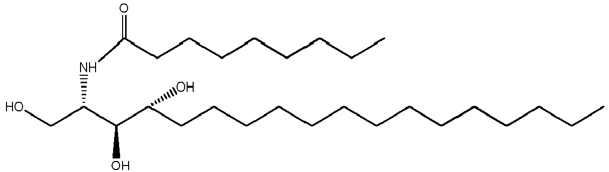

A solution of pelargonic acid (0.56 g, 3.54 mmol) in 5 mL of methyl tetrahydrofuran and triethyl amine (1.09 mL, 7.79 mmol) is added dropwise to a stirred solution of pivaloyl chloride (0.43 g, 3.54 mmol) in 10 mL of methyl tetrahydrofuran at 0°C. After stirring for 1 hour at 0°C and monitoring by TLC that the mixed anhydride is formed, the reaction mixture is heated at room temperature. After that, phyto-sphingosine (1.07 g, 3.36 mmol) and other 5 mL of methyl tetrahydrofuran is added, and the reaction is stirred overnight at room temperature. 300 mL of water is added and stirring is continued for 1 hour. After evaporation of tetrahydrofuran, 20 mL of a solution of water/acetone 9:1 is added. The precipitate is filtered off using a sintered glass filter and washed twice, each one with 20 mL water/acetone 9:1 and another twice with 15 mL acetone for each one. Drying gives 1.07 g of pure compound. The precipitate in the mother liquor is filtered off using a sintered glass filter and washed twice, each one with 15 mL of acetone. Drying gives other 70 mg of pure compound. Yield 74%.

¹H NMR (400 MHz, CDCl₃/MeOD 5:1) δ 4.04 (br m, 1H, H2), 3.75 (dd, J = 11.4, 3.7 Hz, 1H, H1a), 3.64 (dd, J = 11.5, 5.4 Hz, 1H, H1b), 3.49 (br m, 2H, H3 and H4), 2.17 (t, J = 7.6 Hz, 2H, H2'), 1.67 - 1.22 (m, 38H, CH₂), 0.84 (t, J = 6.7 Hz, 6H, 2 CH₃).

¹³C NMR (101 MHz, CDCl₃/MeOD 5:1) δ 174.59, 75.65, 72.42, 61.01, 51.87, 36.48, 33.01, 31.84, 31.75, 29.62, 29.58, 29.28, 29.24, 29.21, 29.08, 25.79, 25.71, 22.59, 22.55, 13.94, 13.91.

### Example 10: Synthesis of N-(1,3,4-trihydroxyoctadecan-2-yl)nonanamide

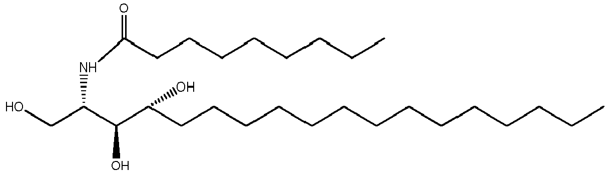

A solution of pelargonic acid (0.56 g, 3.54 mmol) in 5 mL of tetrahydrofuran and triethyl amine (0.59 mL, 4.25 mmol) is added dropwise to a stirred solution of pivaloyl chloride (0.43 g, 3.54 mmol) in 10 mL of tetrahydrofuran at 0°C. After stirring for 1 hour at 0°C and monitoring by TLC that the mixed anhydride is formed, the reaction mixture is heated at room temperature. After that, phyto-sphingosine (1.07 g, 3.36 mmol) solubilized in 5 mL of pyridine is added and the reaction is stirred for 4 hours at room temperature. After evaporation of tetrahydrofuran and pyridine, 20 mL of a solution of water/acetone 9:1 is added. The precipitate is filtered off using a sintered glass filter and washed twice, each one with 20 mL water/acetone 9:1 and other twice with 15 mL acetone for each one. Drying gives 1.06 g of pure compound. The precipitate in the mother liquor is filtered off using a sintered glass filter and washed twice, each one with 10 mL water/acetone 9:1 and other twice with 10 mL of acetone for each one. Drying gives a further 130 mg of pure compound. Yield 77%.

¹H NMR (400 MHz, CDCl₃/MeOD 5:1) δ 4.04 (br m, 1H, H2), 3.75 (dd, J = 11.4, 3.7 Hz, 1H, H1a), 3.64 (dd, J = 11.5, 5.4 Hz, 1H, H1b), 3.49 (br m, 2H, H3 and H4), 2.17 (t, J = 7.6 Hz, 2H, H2'), 1.67 - 1.22 (m, 38H, CH₂), 0.84 (t, J = 6.7 Hz, 6H, 2 CH₃).

^{13C}NMR (101 MHz, CDCl₃/MeOD 5:1) δ 174.59, 75.65, 72.42, 61.01, 51.87, 36.48, 33.01, 31.84, 31.75, 29.62, 29.58, 29.28, 29.24, 29.21, 29.08, 25.79, 25.71, 22.59, 22.55, 13.94, 13.91.

The formation of O-acetylated compound is observed after the second filtration, instead, the procedure carried out in examples 9 and 10 does not generate O-acetylated compound but only pure compound.

## Claims

1. A process for preparing ceramides, comprising the steps of:
a) reacting pivaloyl chloride with an aliphatic acid of formula (I), or a salt thereof, in the presence of a base and a solvent:
R-COOH (I),
wherein R is a straight or a branched C₇-C₂₅ alkyl chain, optionally interrupted by an oxygen atom, optionally containing one or more double bonds, and optionally substituted with one or more hydroxyl groups, thus obtaining a mixed anhydride;
b) reacting said mixed anhydride with phyto-sphingosine, thus obtaining a ceramide.

2. The process according to claim 1, wherein the aliphatic acid of formula (I) is selected from the group consisting of oleic acid, palmitic acid, stearic acid, pelargonic acid, cerotic acid and 12-hydroxystearic acid.

3. The process according to any one of claims 1-2, wherein the base of steps a) and b) is an organic base, preferably selected from the group consisting of tertiary amines, more preferably it is triethyl amine, pyridine, and mixtures thereof.

4. The process according to any one of claims 1-2, wherein the base of steps a) and b) is an inorganic base, preferably selected from the group consisting of cesium carbonate, potassium carbonate and mixtures thereof.

5. The process according to any one of claims 1-4, wherein the solvent of step a) comprises or consists of dichloromethane, ethyl acetate, tetrahydrofuran, methyl tetrahydrofuran, toluene, chloroform, methyl ethyl ketone, and diethyl ether, preferably comprises or consists of tetrahydrofuran and 2-methyltetrahydrofuran, more preferably it is tetrahydrofuran.

6. The process according to any one of claims 1-5, wherein in step a) the ratio of the aliphatic acid of formula (I) to pivaloyl chloride is 1:1.

7. The process according to any one of claims 1-6, wherein in step a) the ratio of the organic base to pivaloyl chloride ranges from 2:1 to 3:1, preferably it is 2.2:1.

8. The process according to any one of claims 1-7, wherein:
- step a) is carried out at a temperature ranging from 0 to 30°C, preferably ranging from 0 to 5°C;
- step b) is carried out at a temperature ranging from 0 to 30°C, preferably ranging from 20 to 28°C.

9. The process according to any one of claims 1-8, wherein:
- step a) is carried out for a time ranging from 1 to 2 hours, preferably for a time 1.5 hours;
- step b) is carried out for a time ranging from 1 to 8 hours, preferably 3 hours.

10. The process according to anyone of claims 1-9, wherein in step b) the ratio of the mixed anhydride to the amino alcohol ranges from 1:0.7 to 1.5:1, preferably it is 1:0.95.

11. The process according to any one of claims 1-10, wherein the solvent is selected from the group consisting of dichloromethane, ethyl acetate, tetrahydrofuran, methyl tetrahydrofuran, toluene, chloroform, methyl ethyl ketone, diethyl ether and mixtures thereof, preferably it is selected from tetrahydrofuran and methyl tetrahydrofuran.

12. The process of claim 1, which is a one-pot process.

13. The process of claims 1 and 12, wherein in step b) phyto-sphingosine is added to the mixed anhydride obtained from step a).

14. The process of claim 1, wherein the hydroxyl present in the alkyl chain of the aliphatic acid (I), if present, is not protected.

15. The process according to any one of the preceding claims, further comprising the purification of the ceramide.
